# EUROPEAN PATENT APPLICATION

(11) **EP 4 644 529 A1**
(43) Date of publication of application: **05.11.2025**
(21) Application number: 24020198.8
(22) Date of filing: 13.06.2024
(51) Int. Cl.: C12N 1/16, C12P 23/00

(54) **RHODOTORULA TORULOIDES BEL 1 YEAST STRAIN AND A METHOD FOR PRODUCING BETA CAROTENE THEREWITH**

(30) Priority: 02.05.2024 BG 11389724
(71) Applicant: "INDEX 11" Joint Stock Company, 4003 Plovdiv (BG)
(72) Inventor: Rangelov, Ivan Rangelov, 4130 Plovdiv (BG); Angelov, Angel Ivanov, 4020 Plovdiv (BG); Kichukov, Todor Atanasov, 4001 Plovdiv (BG)
(74) Representative: Ilarionov, Pavko Jordanov

(57) **Abstract**

The invention relates to a newly isolated yeast strain *Rhodotorula toruloides* Bel 1 and a method for producing beta carotene developed therefrom, which will find application in the food industry and in particular in the feeding of farm animals and fish.

The strain assimilates glucose, sorbose, ribose, sucrose, melibiose, raffinose, inulin, pectin, glycerol, ribitol, mannitol, citric acid, and does not assimilate cellobiose, lactose, starch, erythritol, galactitol, inositol, glucosamine and tartaric acid. Of nitrogenous sources it assimilates inorganic ammonium and nitrate salts, amino acids, does not assimilate urea. Strain grows on nutrient media without the presence of vitamins. The optimum temperature for its development is in the range of 25-30°C, with temperatures above 35°C slowing its development considerably.

A medium formulation has been developed which contains glycerol, vegetable fats, yeast extract, KH₂PO₄, NH₂(SO₄) and MgSO₄. After sterilization and cooling, the culture medium was inoculated with a liquid culture of *Rhodotorula toruloides* Bel 1 strain. The strain has a pronounced capacity for biomass synthesis with a high concentration of carotenes.

## Description

### Technical field

The invention relates to a newly isolated yeast strain *Rhodotorula toruloides* Bel 1 and a method for producing beta carotene developed therefrom, which will find application in the food industry and in the feeding of farm animals and fish.

### Background of the invention

Globally, over 1100 carotenoids with different structures and characteristic biological functions have been discovered from various sources including microbial sources (Yabuzaki, 2017). Carotenoids are essential for human health, but humans cannot synthesize carotenoids, so they must be ingested through a balanced diet (Ram et al., 2020). The structure of carotenoids gives them very specific properties and underlies the basis of several complex biological functions in the human body (Faraone et al., 2020). Several carotenoids have been shown to be precursors of vitamin A and recent studies have reported that carotenoids prevent several serious human health disorders (Bhatt and Patel, 2020).

Many carotenoids are used in a wide range of industrial applications - food, animal feed, cosmetics, medicines, etc., but most of them are chemically synthesized. However, it is believed that natural pigments may be healthier than synthetic pigments and there is a growing demand to phase out synthetic carotenoids in many countries (Mussagy et al., 2019). By optimizing biotechnological methods, carotenoid production can be improved, and overall processing costs can be reduced. In recent years, there has been an increased interest in microbial production of carotenoids, including the isolation of new strains from natural samples, the composition of culture media, and cultivation and purification methods aimed at increasing beta carotene yields. Due to the lipophilic nature of carotenoids, most carotenoids are incorporated into the structural elements of the cell. Traditionally, extraction methods of carotenoids using organic solvents is the most widely used, but an increasing number of studies are applying environmentally friendly and sustainable methods (Saini and Keum, 2018, 2017). In this regard, a variety of research is being conducted including ecological niches for isolation of strain-producers, (Ambati et al., 2019), cultivation and extraction methods (Saini and Keum, 2018) and health effects of carotenoids (Novoveska et al., 2019).

Among industrial microorganisms, bacteria and yeasts have a certain interest to be investigated as beta carotene-producing strains. Fungi and microalgae are also promising in this respect.

In the scientific literature, the method of biosynthesis of beta carotene from *Rhodotorula toruloides* Bel 1 strain, composition of culture medium, culture conditions, isolation and purification of the bioproduct are not known.

### Summary of the invention

It is an object of the invention to isolate a yeast strain and to develop a method for producing beta carotene from yeast biomass comprising a newly isolated strain of *Rhodotorula toruloides* Bel 1, a composition of the culture medium, culture conditions of the strain and isolation of beta carotene which will find application in the food industry, in particular in the feeding of farm animals and fish.

The strain was isolated from natural soil samples of a pine forest around the village of Samegor, Brezovo municipality, Bulgaria, coordinates 42°26'34.0 "N 24°55'36.7 "E. After purification from the microbial association, a pure culture was obtained and identified according to morphological, cultural and biochemical characteristics. After three days of aerobic cultivation in potato dextrose broth (PDB) liquid culture medium at 25°C, the morphology of the cells was as follows: spherical to ovoid in shape with a size of (2-5)×(5-10)µ. The cells are predominantly single and less frequently grouped in pairs. Colonies grown after three days incubation on potato dextrose agar (PDA) medium at 25°C have the following culture characteristics: the colonies are pink to red in colour, their consistency is soft, spreadable, the surface is smooth and shiny, the margins are flat, not striated and the shape is dome shaped. The colonies are 2-3 mm in size. The strain assimilates the following carbon sources: glucose, sorbose, ribose, sucrose, melibiose, raffinose, inulin, pectin, glycerol, ribitol, mannitol, citric acid; and does not assimilate: cellobiose, lactose, starch, erythritol, galactitol, inositol, glucosamine and tartaric acid. Of nitrogenous sources assimilates inorganic ammonium and nitrate salts, amino acids, does not assimilate urea. The strain grows on nutrient media without the presence of vitamins. The optimum temperature for strain development is in the range of 25-30°C, with temperatures above 35°C slowing its development considerably.

The sequences of *Rhodotorula toruloides* Bel 1 strain are as follows:
Internal transcribed spacer 1 
TACCCAAGACAAATGCACAGGGTTAGAAAGTGAGGGTCGGGCTCCA
AGTTAAGTTGGACACCCTCATATTCACTAATGATCCCTTTCCGTAGGT
GAACCTGCGGAAGGATCATTAGTGAATATTAGGGTGTCCAACTTAAC
TTGGAGCCCGACCCTCACTTTCTAACCCTGTGCATTTGTCTTGGGTAG
TAGCTTGCGTCAGCGAGCGAATCCCATTTCACTTACAAACACAAAGT
CTATGAATGTAACAAATTTATAACAAAACAAAACTTTCAACAACGGA
TCTCTTGGCTCTCGCATCGATGAAGAACGCAGCGAAATGCGATACGT
AATGTGAATTGCAGAATTCAGTGAATCATCGAATCTTTGAACGCACC
TTGCGCTCCATGGTATTCCGTGGAGCATGCCTGTTTGAGTGTCATGAA
 
TTCTTCAACCCACCTCTTTCTTAGTGAATCAGGCGGTGTTTGGATTCT
GAGCGCTGCTGGCTTCGCGGCCTAGCTCGCTCGTAATGCATTAGCAT
CCGCAATCGAACTTCGGATTGACTCGGCGTAATAGACTATTCGCTGA
GGATTCTGGTCTCTGACTGGAGCCGGGTAAGGTTAAAGGAGCTACT
AATCCTCATGTCTATCTTGAGATTAGACCTCAAATCAGGTAGGACTA
CCCGCTGAACTTAAGCATATCAATAAGCCGGAGGAAA
Large subunit ribosomal RNA gene

AAAGCGGAGGAAAAGAAACTAACAAGGATTCCCCTAGTAGCGGCGA
GCGAAGCGGAAGAGCTCAAATTTGTAATCTGGCACCTTCGGTGTCC
GAGTTGTAATCTCTAGAAGTGTTTTCCGCGTTGGACCGCACACAAGT
CTGTTGGAATACAGCGGCATAGTGGTGATACCCCATTACACGGTGCG
GACGCCCAGCGCTTTGTGATACACTTTCGACGAGTCGAGTTGTTTGG
GAATGCAGCTCAAATTGGGTGGTAAATTCCATCTAAAGCTAAATATT
GGCGAGAGACCGATAGCGAACAAGTACCGTGAGGGAAAGATGAAAA
GCACTTTGGAAAGAGAGTTAAAAGTACGTGAAATTGTTGGAAGGGA
AACGCTTGAAGTCAGACTTGCTTGCCGGGCTTGCTCGGTTTGCAGGC
CAGCATCAGTTTTCCGGGGCGGATAATGGCAGTTAGAATGTAGCAGT
CTCGGCTGTGTTATAGCTTTCTGCTGGATACGTCCTGGGGGACTGAG
GAACGCAGCGTGCCGTATGGCGAGGGCTTCGGTCCTTTCACGCTTAG
GATGCTGGTGGAATGGCTTTAAACGACCCGTCTTGAAC

The newly isolated strain *Rhodotorula toruloides* Bel 1 has been deposited in the National Bank for Industrial Microorganisms and Cell Culture, Bulgaria, under number **NBIMCC 9110** dated 05.04.2024.

The problem was solved by creating a method for producing beta carotene with *Rhodotorula toruloides* Bel 1 strain and culture medium. The strain has pronounced capabilities for biomass synthesis with high concentration of carotenes.

The created method includes the following sequential operations:
- Preparation of yeast biomass culture medium containing in g/dm³ the following components in weight percent: glycerol (glycerin) from 14.00 to 33.00%; vegetable fat from 56.00 to 73.00%; yeast extract from 8.00 to 11.00%; KH₂PO₄ from 1.80 to 5.20%; (NH₄)₂SO₄ from 1.40 to 2.30%; MgSO₄ from 1.10 to 2.30%. The hydrogen pH of the culture medium was adjusted to 7.00 with NH₄OH;
- sterilisation and cooling of the prepared culture medium;
- preparation of an inoculum of *Rhodotorula toruloides* Bel 1 strain in a bioreactor with a culture medium composed of potato dextrose broth at a temperature of 25-30°C and a duration of 24-48 hours with constant agitation;
- inoculation of the sterilised and cooled yeast biomass culture medium with liquid inoculum culture of *Rhodotorula toruloides* Bel 1 strain.
- Biosynthesis of beta carotene from the culture medium in the previous step at 25-30°C and duration 72-115 hours with constant stirring;
- separation of yeast biomass in a centrifuge at 4000 - 6500 min⁻¹;
- ball mill disintegration of the biomass at room temperature for 25-35 minutes;
- extraction of beta carotene from the yeast biomass using a 1:2:2 mixture of acetone, methanol and chloroform solvents under continuous stirring and under pressure in a nitrogen atmosphere;
- obtaining two phases after extraction, the first phase containing a chloroform/acetone mixture saturated with carotenes and single cell fats and the second phase containing water, acetone, methanol and spent biomass;
- evaporation of the first phase in a thin-layer vacuum evaporator to regenerate the chloroform/acetone mixture and separate the carotenes with the fats as a concentrated oil extract;
- distillation of the second phase in an acetone or methanol recovery column; and
- drying of the spent biomass or biomass after separation in a spray dryer.

The advantages of the established method lie in the isolation from natural samples and the molecular identification of a *Rhodotorula toruloides* Bel 1 strain, which shows the possibility of enhanced beta carotene synthesis on an optimized culture medium. In the development of the culture medium, crude glycerol and vegetable oils were included in its composition, forming the environmental aspect of the invention created and the use of circular economy approaches. An unconditional advantage of the created method is the possibility of complete regeneration of the used extractives, which leads to significant economic benefits and reduces the negative impact on the environment.

### Description of embodiment of the invention

In one exemplary embodiment of the invention, a preferred culture medium composition for the industrial cultivation of a *Rhodotorula toruloides* Bel 1 strain for yeast biomass includes the following components:
- glycerol (glycerin) - 30,5 g/dm³;
- vegetable fat - 62,5 g/dm³;
- yeast extract - 10,5 g/dm³;
- KH₂PO₄ - 4,0 g/dm³;
- (NH₄)₂SO₄ - 1,8 g/dm³;
- MgSO₄ - 1,5 g/dm³;

The hydrogen pH of the culture medium was adjusted to 7.00 with NH₄OH. The prepared culture medium is sterilised and cooled.

The *Rhodotorula toruloides* Bel 1 strain was cultured in a bioreactor with a working volume of 1,5 dm³ of potato dextrose broth at 26°C for 36 hours with constant agitation. At the end of the process, the biomass concentration was 10.80 g/dm³ and the beta-carotene concentration reached 84.00 mg/dm³.

The sterilized and cooled yeast biomass culture medium was inoculated with the liquid inoculum of *Rhodotorula toruloides* strain Bel 1 under the following conditions: medium volume - 1.2 dm³, temperature - 28°C, pH - 6.50, with constant agitation and aeration and duration - 96 hours. Under these conditions, the biomass concentration in the culture medium was 9.80 g/dm³ and the beta carotene concentration was 98.00 mg/dm³.

After biosynthesis of beta carotene, the yeast biomass was separated in a centrifuge at 4500 min⁻¹ for 15 minutes. Disintegration of the biomass was carried out with a ball mill at room temperature for 30 minutes, after which the beta carotene was extracted with a 1:2:2 mixture of solvents acetone, methanol, chloroform under continuous stirring and under pressure in nitrogen medium. After extraction, two phases are obtained, the first phase containing a chloroform/acetone mixture saturated with carotenes and single cell fats and the second phase containing water, acetone, methanol and spent biomass. To regenerate the chloroform/acetone mixture and separate the carotenes as a concentrated oil extract, the first phase is evaporated in a thin layer vacuum evaporator. The aqueous phase is distilled off in an acetone or methanol recovery column. Drying of the extracted biomass or the biomass after separation takes place in a spray dryer at a thermal agent temperature of 230°C.

### References:

Ambati, R.R., Gogisetty, D., Aswathanarayana, R.G., Ravi, S., Bikkina, P.N., Bo, L., Yuepeng, S., 2019. Industrial potential of carotenoid pigments from microalgae: Current trends and future prospects. Crit. Rev. Food Sci. Nutr. 59, 1880-1902.
Bhatt, T., Patel, K., 2020. Carotenoids: Potent to prevent diseases review. Nat. Products Bioprospect. 10, 109-117
Faraone, I., Sinisgalli, C., Ostuni, A., Armentano, M.F., Carmosino, M., Milella, L., Russo, D., Labanca, F., Khan, H., 2020. Astaxanthin anticancer effects are mediated through multiple molecular mechanisms: A systematic review. Pharmacol. Res. 155, 104689
Mussagy, C.U., Winterburn, J., Santos-Ebinuma, V.C., Pereira, J.F.B., 2019. Production and extraction of carotenoids produced by microorganisms. Appl. Microbiol. Biotechnol. 103, 1095-1114.
Novoveska, L., Ross, M.E., Stanley, M.S., Pradelles, R., Wasiolek, V., Sassi, J.F., 2019. Microalgal carotenoids: A review of production, current markets, regulations, and future direction. Mar. Drugs 17, 640-661.
Ram, S., Mitra, M., Shah, F., Tirkey, S.R., Mishra, S., 2020. Bacteria as an alternate biofactory for carotenoid production: A review of its applications, opportunities and challenges. J. Funct. Foods 67, 103867
Saini, R.K., Keum, Y.S., 2017. Progress in Microbial Carotenoids Production. Indian J. Microbiol. 57, 129-130.
Saini, R.K., Keum, Y.S., 2018. Carotenoid extraction methods: A review of recent developments. Food Chem. 240, 90-103.
Yabuzaki, J., 2017. Carotenoids Database: structures, chemical fingerprints and distribution among organisms. Database-the J. Biol. Databases Curation 2017, bax004.

## Claims

1. **Strain *Rhodotorula toruloides* Bel 1,** filed with the National Bank for Industrial Microorganisms and Cell Cultures, Bulgaria, under number **NBIMCC 9110, dated 05.04.2024.**

2. **A method for producing beta carotene with the yeast strain *Rhodotorula toruloides* Bel 1, characterized in that** it comprises the following sequential operations:
- preparation of a yeast biomass culture medium containing in g/dm³ the following components in weight percent: glycerol (glycerin) from 14.00 to 33.00%; vegetable fat from 56.00 to 73.00%; yeast extract from 8.00 to 11.00%; KH₂PO₄ from 1.80 to 5.20%; NH₂(SO₄) from 1.40 to 2.30%; MgSO₄ from 1.10 to 2.30%. The hydrogen pH of the culture medium was adjusted to 7.00 with NH₄OH;
- sterilization and cooling of the prepared culture medium;
- preparation of an inoculum of *Rhodotorula toruloides* Bel 1 strain in a bioreactor with a culture medium composed of potato dextrose broth at a temperature of 25-30°C and a duration of 24-48 hours with constant agitation;
- inoculation of the sterilised and cooled yeast biomass culture medium with liquid inoculum of Rhodotorula toruloides Bel 1 strain.
- biosynthesis of beta carotene from the culture medium in the previous step at 25-30°C and duration 72-115 hours with constant stirring;
- separation of yeast biomass in a centrifuge at 4000 - 6500 min⁻¹;
- mechanical ball mill disintegration of the biomass at room temperature for 25-35 minutes;
- extraction of beta carotene from the yeast biomass using a 1:2:2 mixture of acetone, methanol and chloroform solvents under continuous stirring and under pressure in a nitrogen atmosphere;
- obtaining two phases after extraction, the first phase containing a chloroform/acetone mixture saturated with carotenes and single cell fats and the second phase containing water, acetone, methanol and spent biomass;
- evaporation of the first phase in a thin-layer vacuum evaporator to regenerate the chloroform/acetone mixture and separate the carotenes with the fats as a concentrated oil extract;
- distillation of the second phase in an acetone or methanol recovery column; and
- drying of the spent biomass or biomass after separation in a spray dryer.
